# FASCICULE DE BREVET EUROPEEN

(11) **EP 2 839 834 B1**
(45) Date de publication et mention de la délivrance du brevet: **17.01.2018**
(21) Numéro de dépôt: 14447008.5
(22) Date de dépôt: 18.08.2014
(51) Int. Cl.: A61K 31/60, A61K 8/06, A61K 36/87, A61K 8/44, A61P 17/00

(54) **Composition dermatologique à partir de cellules végétales souches et d'un dérivé d'acide salicylique.**
Dermatologische Zusammensetzung enthaltend planzliche Stammzellen und ein Salizylsäurederivat.
Dermatological composition comprising plant stem cells and a derivate of salicylic acid.

(30) Priorité: 19.08.2013 EP 13447014
(43) Date de publication de la demande: 25.02.2015
(73) Titulaire: Ennamany, Rachid, 33800 Bordeaux (FR)
(72) Inventeur: Ennamany, Rachid, F-33800 Bordeaux (FR); Guillaume, Bruno, F-33800 Bordeaux (FR)
(74) Mandataire: Powis de Tenbossche, Roland

(56) Documents cités:
- EP-A1- 0 585 170
- WO-A1-03/084553
- WO-A2-03/070161
- WO-A2-03/077881
- US-A- 5 001 156
- US-A1- 2011 165 136

## Description

La présente invention a pour objet une composition pour la retructuration de l'épiderme ou pour le traitement de dermatose par application topique comprenant (a) une dispsersion huile dans eau ou étant adaptée pour former une dispersion huile dans eau après ajout d'une quantité d'eau, et (b) une quantité efficace d'au moins un dérivé d'acide salicylique de formule I ou d'un mélange de dérivés d'acide salicylique de formule I 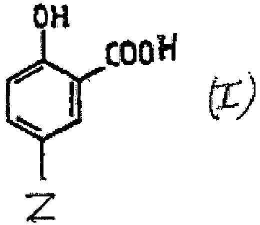 dans laquelle Z est un atome d'hydrogène ou un radical de formule 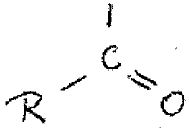 avec R un radical alkyle linéaire ou ramifié avec 1 à 18 atomes de carbone, en particulier avec de 6 à 14 atomes de carbone,
ladite quantité efficace étant au moins supérieure à 0,5% au poids sec de la composition.

On connaît par le document EP0585170 une composition pour le traitement de l'acné contenant au moins un dérivé ammonium d'acides salicyliques particulier. Même si cette composition s'est avérée être relativement efficace, elle ne permettait pas un traitement optimal de l'épiderme, à la fois d'un point de vue efficacité de traitement et rapidité du traitement.

On connaît également par le document WO03/077881 un procédé de production de phytoalexines et une composition pour application topique contenant un broyat de cellules végétales dédifférentiées et élicitées en culture in vitro pour synthétiser des phytoalexines.

Les exemples 10 et 11 de ce document décrivent des dispersions de cellule vignes élicitées (broyées ou non) dans une base cosmétique contenant 0,2% d'acide salicylique.

Une telle teneur en acide salicylique n'est pas efficace pour la restructuration de l'épiderme, ni pour le traitement de dermatose par application topique.

On connaît également par le document WO 03/084553 une composition à administration topique comprenant en tant principe actif des proantocyanidines d'origine végétale sous forme d'un extrait. Selon ce document, les proanthocyanidines auraient une actions protectrices contre les radicaux libres et une action de restauration de la peau. La composition peut comprendre de l'acide salicylique.

Par le document US2011/0165136, on connaît une composition végétale pour le traitement du psoriasis et autres troubles de la peau, la composition comprenant un mélange d'extraits ou huiles de plusieurs plantes.

Les documents WO03/070161 et US5001156 décrivent respectivement des compositions de soin comprenant de l'acide salicylique et des dérivés ammonium de l'acide salicylique.

Les inventeurs ont maintenant découvert qu'il était possible d'obtenir un traitement efficace et rapide de la dermatose, en particulier de dermatoses inflammatoires par application topique d'une composition comprenant (a) une dispsersion huile dans eau ou étant adaptée pour former une dispersion huile dans eau après ajout d'une quantité d'eau, et (b) une quantité efficace d'au moins un dérivé d'acide salicylique de formule I ou d'un mélange de dérivés d'acide salicylique de formule I 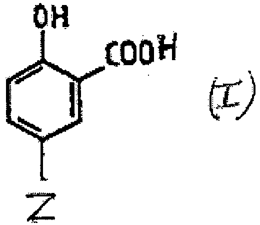 dans laquelle Z est un atome d'hydrogène ou un radical de formule III : 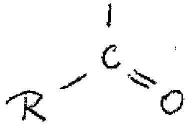 avec R un radical alkyle linéaire ou ramifié avec 1 à 18 atomes de carbone, en particulier avec de 6 à 14 atomes de carbone,
ladite quantité efficace étant au moins supérieure à 0,5% au poids sec de la composition,
ladite composition comprenant en outre au moins 0,5% en poids de cellules végétales dédifférenciées et élicitées de vigne ou d'un broyat de cellules végétales dédifférenciées et élicitées de vigne sous forme sèche par rapport au poids total de la composition, lesdites cellules végétales dédifférenciées et élicitées de vigne ou ledit broyat de cellules végétales dédifférenciées et élicitées de vigne contenant au moins 0,5% en poids de stilbènes par rapport au poids sec des cellules (dans le cas d'un broyat, la teneur de plus de 0,5% en poids de stibènes est une teneur calculée par rapport au poids des cellules avant broyage).

De façon avantageuse, le rapport en poids entre d'une part, le poids sec de dérivé d'acide salicylique ou le mélange de dérivés salicylique, et d'autre part, le poids sec de cellules végétales dédifférenciées et élicitées ou d'un broyat de telles cellules est compris entre 1:50 et 50:1, avantageusement entre 1:25 et 40:1, de préférence entre 1:1 et 30:1.

De préférence, la composition comprend en outre au moins un polymère silicone ou mélange de polymère silicone, le rapport en poids entre d'une part, le poids sec de dérivé d'acide salicylique de formule I et le mélange de dérivés d'acide salicylique de formule I, et d'autre part, le polymère silicone ou mélange de polymères silicone étant compris entre 1:1 et 1:95, avantageusement entre 1:10 et 1: 50.

Selon des formes de réalisation avantageuses d'une composition suivant l'invention, la composition présente une ou plusieurs des particularités suivantes:
- la composition comprend au moins 2% en poids, avantageusement au moins 4% en poids de polymère silicone réticulé possédant des greffons hydrophile polypropylène glycol et/ou de polyéthylène glycol, par rapport au poids sec de la composition, et/ou
- la composition comprend une phase huile dans eau comprenant au moins le dérivé d'acide salicylique de formule I ou le mélange de dérivés d'acide salicylique de formule I dispersée dans une phase à base de silicone, et/ou
- les cellules végétales dédifférenciées et élicitées de vigne, avant leur broyage éventuel, comprennent au moins 0,2% en poids de dérivé(s) d'acide salicylique de formule I, telle que défini(s) à la revendication 1, et/ou
- la composition contient de 0,8% en poids à 5% en poids d'acide salicylique, par rapport au poids sec de la composition, et/ou
- la composition comprend des cellules végétales dédifférenciées élicitées de vigne non broyées, et/ou
- la composition comprend un broyat de cellules végétales dédifférenciées élicitées de vigne, ledit broyat comprenant au moins 95% en poids, avantageusement au moins 97% en poids des cellules végétales dédifférenciées élicitées de vigne avant leur broyage, et/ou
- la composition comprend en outre une ou des vitamines B, en particulier au moins de la vitamine B3, et/ou
- la composition se présente sous la forme d'une double émulsion dont les goutellettes à base d'eau et d'huile sont dispersées dans une phase à base de silicone et présentent une taille moyenne en nombre inférieure à 200µm, avantageusement comprise entre 50µm et 150µm, et/ou
- les gouttelettes à base d'eau et d'huile contiennent le dérivé d'acide salicylique de formule I ou un mélange de dérivés d'acide salicylique de formule I, et un composé basique pour régler le pH des gouttelettes entre 4 et 8, avantageusement entre 4,5 et 7, de préférence entre 4,5 et 6,5, et/ou
- les gouttelettes à base d'eau et d'huile comprennent de l'urée et/ou de la bétaine, et/ou
- les gouttelettes à base d'eau et d'huile comprennent au moins un agent tensio-actif et/ou un alcool, et/ou
- la phase silicone comprend un mélange (a) de silicone(s) volatile(s) dont la pression de vapeur est comprise entre 1Pa et 2000Pa à 25°C et dont la viscosité à 25°C est comprise entre 0,1 et 10 centistokes, et (b) de silicone(s) linéaire(s) réticulé(s), le rapport en poids silicone(s) volatile(s) / silicone(s) linéaire(s) réticulé(s) étant compris entre 0,1 et 3, avantageusement entre 0,1 et 1, et/ou
- la composition se présente sous la forme d'une composition prête à l'application topique contenant de 0,5% en poids à 5% en poids d'un dérivé d'acide salicylique de formule I, avantageusement de 0,7 à 3 % en poids, de préférence de 0,9 à 2,5% en poids, par rapport au poids total de la composition.

Par dérivés de l'acide salicylique de formule I avec Z comme défini dans la revendication 1, on entend également les sels, les esters, etc de l'acide. A titre d'exemple, on peut citer le sel de sodium, les sels d'ammonium de l'acide salicylique de formule I. Les sels d'ammonium de l'acide salicylique répondent avantageusement à la formule II suivante : 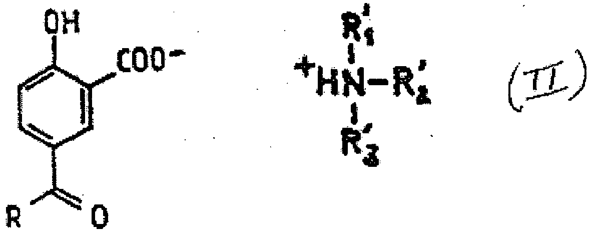 avec R un radical alkyle linéaire ou ramifié avec 1 à 18 atomes de carbone, en particulier avec de 6 à 14 atomes de carbone, et avec R₁',R₂' et R₃', identiques ou différents, représentant un radical alkyle ou hydroalkyle, linéaire ou ramifié, avec de 1 à 18 atomes de carbone, par exemple les radicaux méthyle, éthyle, hexyle, octyle, hydroxyéthyle, hydroxypropyle, etc.

L'invention a encore pour objet l'utilisation de cellules végétales dédifférenciées et élicitées ou d'un broyat de cellules végétales dédifférenciées et élicitées sous forme sèche par rapport au poids total de la composition, lesdites cellules végétales dédifférenciées et élicitées ou ledit broyat contenant au moins 0,5% en poids de stilbènes par rapport au poids sec des cellules, dans la préparation d'une composition pour la restructuration de l'épiderme ou pour le traitement de dermatose par application topique comprenant (a) une dispsersion huile dans eau ou étant adaptée pour former une dispersion huile dans eau après ajout d'une quantité d'eau, et (b) une quantité efficace d'au moins un dérivé d'acide salicylique 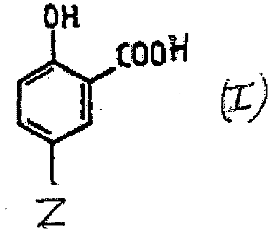 de formule I ou d'un mélange de dérivés d'acide salicylique de formule I
dans laquelle Z est un atome d'hydrogène ou un radical de formule 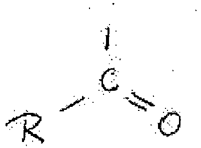 avec R un radical alkyle linéaire ou ramifié avec 1 à 18 atomes de carbone, en particulier avec de 6 à 14 atomes de carbone,
ladite quantité efficace étant au moins supérieure à 0,5% au poids sec de la composition.

La composition suivant l'invention comprend avantageusement des composés hydratant, tensioactifs, de l'huile et des polymères silicone.

A titre de composés hydratant, on peut citer l'urée, les glycols, en particulier, la glycérine ou glycérol, les glycols, le sorbitol, les polyalcools, le propylène glycol, des facteurs hydratants naturels, un extrait d'Aloès vera, des acides aminés, des acides carboxyliques, leurs mélanges et dérivés de ceux-ci. De préfrence, la composition comprend un mélange d'hydratants. L'hydratant ou mélange d'hydratants est présent à raison de moins de 10% en poids, avantageusement à raison de 1 à 8% en poids des compositions topiques selon l'invention (prêtes à l'emploi - poids total de la composition).
A titre d'exemple particulier et préféré, la composition comprend au mois un mélange d'urée et de glycérine ou glycérol, de préférence avec un rapport en poids urée/glycérine compris entre 1:1 et 10:1, en particulier entre 6:1 et 2:1.

A titre d'agent tensioactif, la composition selon l'invention peut comprendre un agent, mais de préférence un mélange d'agents tensioactifs.
On peut citer comme agent tensioactif les alcools gras ayant 12 à 30 atomes de carbone, comme par exemple les alcools laurylique, myristylique, cétylique, stéarylique, cétéarylique, oléique, béhinique, arachidylique et béhénylique, l'hexyldécanol, le 2-octyldodécanol, et leurs mélanges; les acides gras ayant 8 à 30 atomes de carbone, comme par exemple l'acide palmitique, l'acide stéarique, l'acide béhénique ; les esters gras de glycérol, comme par exemple le glycéryl stéarate ; les dérivés oxyéthylénés de ces alcools gras, acides gras et esters gras de glycérol, comportant 2 à 8 groupes oxyde d'éthylène, et leurs mélanges. Cet alcool est présent dans la composition avantageusement à raison de moins de 10% en poids, de préférence de 1 à 6% en poids dans les compositions topiques selon l'invention prêtes à l'emploi (poids total).
A titre d'autres agents tensioactifs, on peut citer les alkylpolyglucosides, de préférence ceux contenant un groupe alkyle comportant de 6 à 30 atomes de carbone, et de préférence de 8 à 22 atomes de carbone, en particulier de 10 à 16 atomes de carbone, et contenant un groupe hydrophile (glucoside) comprenant de préférence 1 ,2 à 3 unités saccharide. Comme alkylpolyglucosides particuliers, on peut citer les alkyiglucosides, et par exemple le décyl glucoside ou alkyl avec de 9 à 11 atomes de carbone -polyglucoside, comme le produit commercialisé sous la dénomination MYDOL 10 ® par la société Kao Chemicals, sous la dénomination PLANTAREN 2000 UP ® par la société Cognis, sous la dénomination ORAMIX NS 10 ® par la société Seppic, et sous la dénomination MACKOL DG ® par la société Rhodia; le caprylyl/capryl glucoside comme le produit commercialisé sous la dénomination ORAMIX CG 1 10 ® par la Société Seppic ; le laurylglucoside comme les produits commercialisés sous les dénominations PLANTAREN 1200 N ® et PLANTACARE 1200 ® par la société Cognis ; et le coco-glucoside comme le produit commercialisé sous la dénomination PLANTACARE 818/UP ® par la société Cognis. De tels glucosides sont par exemple obtenus par condensation de glucose et d'alcools gras linéaires ou ramifiés.
De préférence, l'alkylpolyglucoside de la composition est choisi dans le groupe des alkylglucosides et plus particulièrement dans le groupe constitué du coco-glucoside, du décylglucoside, cétyl stéaryle glucoside et du laurylglucoside, ainsi que de leurs mélanges.

En tant qu'acide salicylique ou dérivé de celui-ci, on peut citer:
l'acide salicylique de formule I avec Z = H (AC1), les sels de celui-ci (par exemple le sel de sodium de l'acide salicylique AC1Na, le triméthylammonium de l'acide salicyilique AC1N+),
l'acide de formule I avec Z = (C₁₁H₂₃)-CO (AC2), les sels de celui-ci (par exemple le sel de sodium de l'acide salicylique AC2Na, le triméthylammonium de l'acide salicyilique AC2N+);
l'acide de formule I avec Z = (C₁₃H₂₅)-CO (AC3), les sels de celui-ci (par exemple le sel de sodium de l'acide salicylique AC3Na, le triméthylammonium de l'acide salicyilique AC3N+);
l'acide de formule I avec Z = (C₁₅H₃₁)-CO (AC4), les sels de celui-ci (par exemple le sel de sodium de l'acide salicylique AC4Na, le triméthylammonium de l'acide salicyilique AC4N+).

La composition selon l'invention contient une ou plusieurs huiles végétales. On entend par "huile" tout composé lipophile, non ionique, insoluble dans l'eau et liquide à température ambiante (25 °C) et sous pression atmosphérique (760 mm de Hg, soit 1,013 10⁵ Pa). Par insoluble dans l'eau, on entend au sens de la présente invention un composé dont la solubilité à pH spontané dans l'eau à 25 °C et à pression atmosphérique est inférieure à 1 %, et de préférence inférieure à 0,5 % en poids. En particulier, on entend par "huile végétale" une huile extraite d'une espèce appartenant au règne végétal. L'huile végétale ou les huiles végétales utilisées selon l'invention sont différentes des alcanes linéaires volatils tels que définis ci-avant et sont choisies parmi les huiles végétales habituellement employées dans le domaine cosmétique.
A titre d'exemple d'huile végétale utilisable dans les compositions de l'invention, on peut citer : - l'huile d'amande douce, - l'huile d'argan, - l'huile d'avocat, - l'huile d'arachide, - l'huile de camélia, - l'huile de carthame, - l'huile de calophyllum, - l'huile de colza, - l'huile de coprah, - l'huile de coriandre, - l'huile de courge, - l'huile de germes de blé, - l'huile de jojoba ou cire liquide de jojoba, - l'huile de lin, - l'huile de macadamia, - l'huile de germes de maïs, - l'huile de noisette, - l'huile de noix, - l'huile de vernonia, - l'huile de noyau d'abricot, - l'huile d'olive, - l'huile d'onagre, - l'huile de palme, - l'huile de passiflore, - l'huile de pépins de raisin, - l'huile de rosier, - l'huile de ricin, - l'huile de seigle, - l'huile de sésame, - l'huile de son de riz, - l'huile de soja, et - l'huile de tournesol.
Les huiles végétales selon l'invention n'ont en général pas subi de transformation chimique après extraction. Parmi les huiles végétales citées ci-dessus, on utilise de préférence l'huile d'olive, l'huile d'argan, l'huile d'avocat, l'huile de colza, l'huile de jojoba ou cire liquide de jojoba, l'huile de soja, l'huile de tournesol, et de manière plus préférée l'huile d'avocat, l'huile de jojoba ou cire liquide de jojoba. De préférence, l'huile ou les huiles végétales sont présentes dans la composition dans une quantité variant de 0,3 à 30%, plus préférentiellement dans une quantité variant de 1 à 20%, et mieux encore dans une quantité variant de 3 à 15% en poids, par rapport au poids total de la composition.

La phase à base de silicone (avantageusement présente dans la composition suivant l'invention) comprend avantageusement un solvant pour les composés silicone, de préférence un solvant silicone. Le solvant est avantageusement choisi parmi les silicones linéaires, les silicones cycliques, les hydrocarbures et leurs mélanges. De préférence, la teneur en hydrocarbures est de moins de 10% de la quantité totale de solvant pour la phase silicone.

Parmi les siliconés linéaires, on peut citer notamment les polydiméthylsiloxanes volatiles et non volatiles et en particulier ceux de formule :

(CH₃)₃-Si-O-[Si(CH₃)₂-O]ₙ-Si-(CH₃)₃

dans laquelle n est un entier de 0 à 7, et de préférence de 0 à 5.

Parmi les composés siliconés cycliques, on peut citer notamment ceux de formule générale :

-(Si(CH₃)₂-O)ₙ-

dans laquelle n est un entier de 3 à 6.

Les silicones linéaires et cycliques sont par exemple celles commercialisées par Dow Corning Corporation, Shin-Etsu, Siltech Corp. et Momentive Performance Materials ou GE Silicones. De telles silicones sont par exemples les silicones Dow Corning 244 ®, 245 ®, 344 ® et 200* Fluids.

Parmi les hydrocarbures, on peut citer notamment les hydrocarbures linéaires ou ramifiés comprenant de 5 à 40 atomes de carbone, et de préférence de 8 à 20 atomes de carbone tels que le pentane, l'hexane, l'heptane, le décane, le dodécane, le tétradécane, le tridécane et les isoparaffines comprenant 8 à 20 atomes de carbone telles que l'isododécane, l'isohexadécane.

A titre de solvants préférés pour la phase silicone de compositions suivant l'invention, on citera les cyclomethicones, et en particulier le cyclopentasiloxane, le cyclohexasiloxane et leurs mélanges.

Le ou les solvants présents dans la phase silicone représentent avantageusement 40 à 75% en poids de la phase silicone.

La phase silicone comprend avantageusement un polymère silicone non réticulé et /ou un polymère silicone réticulé, avantageusement au moins un polymère silicone réticulé et un polymère silicone non réticulé.

Parmi les polymères silicone non réticulés, on citera les composés silicone émulsifiants, en particulier des dérivés polyol de diméthicone, de préférence des copolyols diméthicone. De tels émulsionnants sont décrits aux pages 3 et 4 du document FR2924937 et pages 8 à 10 de EP2186544. A titre d'exemples particuliers, on citera les diméthicones de polyéthylène glycol et de polypropylène glycol, en particulier les alkyl diméthicone copolyols de formule générale (IV) : 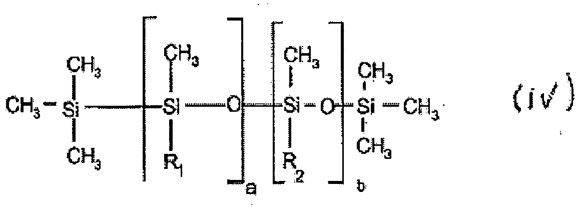 dans lesquelles :
R₁ désigne un groupement alkyle linéaire ou ramifié avec de 1 à 20 atomes de carbone, et de préférence avec 12 à 18 atomes de carbone ;
R₂ désigne le groupement :--CₙH₂ₙ--(-OC₂H₄-)ₓ--(-OC₃H₆-)_{y}-O-R₃,
R₃ désigne un atome d'hydrogène ou un radical akyle linéaire ou ramifié comportant de 1 à 12 atomes de carbone ;
a est un nombre entier allant de 1 à environ 500 ;
b désigne un nombre entier allant de 1 à environ 500 ;
n est un nombre entier allant de 2 à 12 et de préférence 2 à 5 ;
x désigne un nombre entier allant de 1 à environ 50 et de préférence de 1 à 30 ;
y désigne un nombre entier allant de 0 à environ 49 et de préférence 0 à 29 .
Parmi les émulsionnants alkyldimethicone copolyols de formule (IV), on peut citer le CETYL PEG/PPG-10/1 DIMETHICONE, le mélange CETYL PEG/PPG-10/1 DIMETICONE AND DIMETHICONE (nom INCI) comme le produit vendu sous le nom commercial ABIL EM90 ® par la société GOLDSCHMIDT ou bien le mélange (POLYGLYCERYL-4-STEARATE and CETYL PEG/PPG-10 (AND) DIMETHICONE et HEXYL LAURATE) comme le produit vendu sous le nom commercial ABIL WE09 ® par la même société,
le PEG-18/PPG-18 DIMETHICONE et le mélange CYCLOPENTASILOXANE et PEG-18/PPG- 18 DIMETHICONE (nom INCI) tel que le produit vendu par la société Dow Corning sous la dénomination commerciale Silicone DC 5225 C ® ou KF-6040 ® de la société Shin Etsu, ainsi que les mélanges suivants :
   un mélange de PEG-18/PPG-18 DIMETHICONE et CETYL PEG/PPG-10/1 DIMETHICONE et plus particulièrement un mélange de (CYCLOPENTASILOXANE et PEG-18/PPG-18 DIMETHICONE) et de CETYL PEG/PPG-10/1 DIMETICONE et DIMETHICONE ou de (POLYGLYCERYL-4-STEARATE et CETYL PEG/PPG- 25 et DIMETHICONE (AND) HEXYL LAURATE).

Les cellules végétales dédifférenciées et élicitées de vigne présentes dans les compositions suivant l'invention, éventuellement sous forme d'un broyat sont avantageusement du type décrit dans WO 03/077881. Ces cellules et le broyat de celles-ci sont riches en stilbène, par exemple avec une teneur de 0,5 à 3% en poids, voire plus, telle qu'une teneur de 0,8% en poids, 1%, 1,5 et 2% en poids.

La composition peut comprendre un ou plusieurs additifs supplémentaires, tels que parfums, antioxydants, vitamine, alcools, etc.
Parmi les vitamines, on peut citer les vitamines B3, B6, B9 et leurs mélanges. Les vitamines sont par exemple présents à raison de 0,2 à 2% du poids total de la composition.

Des compositions suivant l'invention ont été préparées en utilisant les composés suivants :
Eau (Aqua) ; Alcool cétylique, Cétylstéaryle glucoside, urée, glycérine, l'acide salicylique de formule I avec Z = H (AC1), le sel de sodium de l'acide salicylique AC1Na, le triméthylammonium de l'acide salicyilique AC1N+, l'acide de formule I avec Z = (C₁₁H₂₃)-CO (AC2), le sel de sodium de l'acide salicylique AC2Na, le triméthylammonium de l'acide salicyilique AC2N+; l'acide de formule I avec Z = (C₁₃H₂₅)-CO (AC3), le sel de sodium de l'acide salicylique AC3Na, le triméthylammonium de l'acide salicyilique AC3N+; l'acide de formule I avec Z = (C₁₅H₃₁)-CO (AC4), le sel de sodium de l'acide salicylique AC4Na, le triméthylammonium de l'acide salicyilique AC4N+, cellules végétale dédifférenciées et élicitées de vigne (teneur en stilbène de 1% en poids par rapport au poids sec des cellules et teneur en acide salicylique ou dérivé d'acide salicylique de formule I d'au moins 0,4% en poids par rapport au poids sec) CV1, broyat de cellules de vigne CV1, la vitamine PP ou B3 (niacinamide), octyldodécanol, argan, huile d'olive vierge, l'huile de pépin de raisin, l'huile de Jojoba, le PEG/PPG 19/19 diméthicone (polymère silicone modifié hydrophile), le cyclopentasiloxane, le copolymère PEG 10 dimethicone (polymère silicone réticulé modifié hydrophile) .

Pour la préparation de compositions suivant l'invention, on réalise d'abord une phase aqueuse contenant l'acide salicylique ou le dérivé de cet acide et les cellules végétales dédifférenciées et élicitées de vigne. On prépare ensuite une phase huileuse en mélangeant l'octyldodécanol et l'huile ou les huiles. La phase huileuse est éventuellement chauffée pour assurer une meilleure fluidité et une plus faible viscosité. Avantageusement, la phase aqueuse et la phase huileuse sont portées sensiblement à une même température (par exemple de 25 à 50°C) avant d'être mélangées vigoureusement entre elles.

Cette dispersion huile dans eau est avantageusement dispersée dans une phase silicone. Pour ce faire, la phase silicone est préparée en mélangeant le PEG/PPG 19/19 diméthicone (polymère silicone modifié hydrophile), le cyclopentasiloxane et le copolymère PEG 10 dimethicone (polymère silicone réticulé modifié hydrophile), par exemple à une température comprise entre 20 et 50°C. La dispersion huile dans eau est ensuite ajoutée à cette phase silicone, tout en étant soumise à un mélangeage.

Les tableaux suivants donnent des compositions suivant l'invention, données à titre d'exemple uniquement:

### Composition 1 (dispersion huile dans eau)

| Phase | composé | % en poids dans la phase | fraction massique des phases dans la composition | % en poids dans la composition finale |
|---|---|---|---|---|
| Aqueuse | Aqua | 69 | | 48,3 |
| Aqueuse | urée | 10 | | 7 |
| Aqueuse | glycérine | 3,5 | | 2,45 |
| Aqueuse | alcool cétylique | 5,5 | | 3,85 |
| Aqueuse | cétylstéaryle glucoside | 8 | | 5,6 |
| Aqueuse | CV1 | 0,5 | | 0,35 |
| Aqueuse | AC1 | 3,5 | 70 | 2,45 |
| Aqueuse | **total** | **100** | | |
| Huile | octyldodécanol | 80 | | 24 |
| Huile | argan | 20 | | 6 |
| Huile | **total** | **100** | 30 | |

### Composition 2 (dispersion huile dans eau)

| Phase | composé | % en poids dans la phase | fraction massique des phases dans la composition | % en poids dans la composition finale |
|---|---|---|---|---|
| Aqueuse | Aqua | 69 | | 48,3 |
| Aqueuse | urée | 10 | | 7 |
| Aqueuse | glycérine | 3,5 | | 2,45 |
| Aqueuse | alcool cétylique | 5 | | 3,5 |
| Aqueuse | cétylstéaryle glucoside | 8 | | 5,6 |
| Aquesue | Vitamine B3 | 0,5 | 70 | 0,35 |
| Aqueuse | CV1 | 0,5 | | 0,35 |
| Aqueuse | AC1 | 3,5 | | 2,45 |
| Aqueuse | **total** | **100** | | |
| Huile | octyldodécanol | 80 | | 24 |
| Huile | argan | 20 | | 6 |
| Huile | **total** | **100** | 30 | |

### Composition 3 (dispersion huile dans eau)

| Phase | composé | % en poids dans la phase | fraction massique des phases dans la composition | % en poids dans la composition finale |
|---|---|---|---|---|
| Aqueuse | Aqua | 69 | | 48,3 |
| Aqueuse | urée | 5 | | 3,5 |
| Aqueuse | glycine bétaine | 5 | | 2,5 |
| Aqueuse | glycérine | 3,5 | | 2,45 |
| Aqueuse | alcool cétylique | 5 | | 3,5 |
| Aqueuse | cétylstéaryle glucoside | 8 | 70 | 5,6 |
| Aquesue | Vitamine B3 | 0,5 | | 0,35 |
| Aqueuse | broyat de CV1 | 0,5 | | 0,35 |
| Aqueuse | AC1Na | 3,5 | | 2,45 |
| Aqueuse | **total** | **100** | | |
| Huile | octyldodécanol | 80 | | 24 |
| Huile | huile d'olive | 10 | | |
| Huile | argan | 10 | 30 | 6 |
| Huile | **total** | **100** | | |

### Composition 4 (dispersion huile dans eau)

| Phase | composé | % en poids dans la phase | fraction massique des phases dans la composition | % en poids dans la composition finale |
|---|---|---|---|---|
| Aqueuse | Aqua | 69 | | 48,3 |
| Aqueuse | urée | 10 | | 7 |
| Aqueuse | glycérine | 3,5 | | 2,45 |
| Aqueuse | alcool cétylique | 5 | | 3,5 |
| Aqueuse | cétylstéaryle glucoside | 8 | | 5,6 |
| Aquesue | Vitamine B3 | 0,5 | 70 | 0,35 |
| Aqueuse | broyat de CV1 | 0,5 | | 0,35 |
| Aqueuse | AC1N+ | 3,5 | | 2,45 |
| Aqueuse | **total** | **100** | | |
| Huile | octyldodécan ol | 80 | | 24 |
| Huile | huile d'olive | 10 | 30 | |
| Huile | huile de Jojoba | 5 | | |
| Huile | huile de pépin de raisin | 5 | | 6 |
| Huile | **total** | **100** | | |

### Compositions 5 à 8

Les compositions 5 à 8 correspondent aux compositions 1 à 4, si ce n'est que l'on a changé les composés AC1, AC1Na et AC1N+ respectivement par AC2, AC2Na et AC2N+.

### Compositions 9 à 12

Les compositions 9 à 12 correspondent aux compositions 1 à 4, si ce n'est que l'on a changé les composés AC1, AC1Na et AC1N+ respectivement par AC3, AC3Na et AC3N+.

### Compositions 13 à 16

Les compositions 13 à 16 correspondent aux compositions 1 à 4, si ce n'est que l'on a changé les composés AC1, AC1Na et AC1N+ respectivement par AC4, AC4Na et AC4N+.

### Compositions avec phases silicones

Diverses phases silicones ont été préparées. Ces phases silicones sont données dans le tableau suivant :

| Phase silicone (PS) | PEG/PPG 19/19 diméthicone % en poids | cyclopentasiloxane % en poids | PEG 10 diméthicone % en poids |
|---|---|---|---|
| PS1 | 50 | 50 | |
| PS2 | 10 | 60 | 30 |
| PS3 | | 60 | 40 |
| PS4 | 50 | 40 | 10 |
| PS5 | 30 | 50 | 20 |
| PS6 | 25 | 50 | 25 |
| PS7 | 20 | 50 | 30 |

### Compositions 1 à 16 avec phase silicone

Les compositions 1 à 16 ont été mélangées à une phase silicone PS1 à PS7. La quantité de phase silicone par rapport à la quantité de dispersion huile dans eau (compositions 1 à 16) a été adaptée pour que la teneur en phase silicone de la composition finale soit respectivement 15% en poids, 20% en poids, 25% en poids, 30% en poids, 40% en poids, 50% en poids et 60% en poids.

D'autres compositions selon l'invention ont été préparées de manières similaires aux compositions 1 à 16, avec ou sans phase silicone, mais en faisant varier la teneur en acide salicylique ou en dérivé de formule I, par exemple entre 2 et 5% par rapport à la composition finale.

### Tests d'efficacité

Des sujets volontaires porteurs de lésions érythémato-squameuses de psoriasis ont été traités par application topique (de manière journalière pendant 7 jours) sur les lésions d'une composition constituées à raison de 60% en poids de la composition huile dans eau n°3 et à raison de 40% en poids de phase silicone PS5. A titre comparatif, deux autres patients ont été traités par la même composition, si ce n'est que la dispersion huile dans eau ne contenait pas de broyat de cellule de vigne.

Les malades présentait un score total en "érythème + squames + infiltrations" supérieur à 5, et ne suivaient pas de traitement pour le psoriasis, ni traitement à base d'aspirine.

L'intensité des critères d'évaluation est codée de 0 à 3 (0= nulle, 1= légère, 2= moyenne, 3= forte).

Après deux semaines de traitements, les lésions ont été observées:

En utilisant la composition selon l'invention, on a observé une diminution marquée de l'érythème, des squames et de l'infiltration, et l'absence quasi totale d'irritation. Aucune allergie n'a été observée. Avec la composition comparative, on n'a observé sensiblement aucune diminution de l'érithène, squames et infiltration.

La composition suivant l'invention permet donc un traitement efficace et rapide de dermatose, par rapport à la composition comparative.

## Revendications

1. Composition topique pour son utilisation dans la restructuration de l'épiderme ou dans le traitement de dermatose par application topique comprenant (a) une dispsersion huile dans eau ou étant adaptée pour former une dispersion huile dans eau après ajout d'une quantité d'eau, et (b) une quantité efficace d'au moins un dérivé d'acide salicylique de formule I ou d'un mélange de dérivés d'acide salicylique de formule I 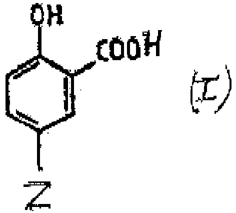 dans laquelle Z est un atome d'hydrogène ou un radical de formule 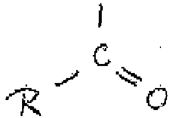 avec R un radical alkyle linéaire ou ramifié avec 1 à 18 atomes de carbone, en particulier avec de 6 à 14 atomes de carbone,
ladite quantité efficace étant au moins supérieure à 0,5% au poids sec de la composition,
**caractérisée en ce qu'**elle comprend en outre au moins 0,5% en poids de cellules végétales dédifférenciées et élicitées de vigne ou d'un broyat de cellules végétales dédifférenciées et élicitées de vigne sous forme sèche par rapport au poids total de la composition, lesdites cellules végétales dédifférenciées et élicitées de vigne ou ledit broyat broyat de cellules végétales dédifférenciées et élicitées de vigne contenant au moins 0,5% en poids de stilbènes par rapport au poids sec des cellules.

2. Composition pour son utilisation suivant la revendication 1, **caractérisée en ce que** le rapport en poids entre d'une part, le poids sec de dérivé d'acide salicylique ou le mélange de dérivés salicylique, et d'autre part, le poids sec de cellules végétales dédifférenciées et élicitées de vigne ou d'un broyat de telles cellules végétales dédifférenciées et élicitées de vigne est compris entre 1:50 et 50:1, avantageusement entre 1:25 et 40:1, de préférence entre 1:1 et 30:1.

3. Composition pour son utilisation suivant la revendication 1 ou 2, **caractérisée en ce qu'**elle comprend en outre au moins un polymère silicone ou mélange de polymère silicone, le rapport en poids entre d'une part, le poids sec de dérivé d'acide salicylique de formule I et le mélange de dérivés d'acide salicylique de formule I, et d'autre part, le polymère silicone ou mélange de polymères silicone étant compris entre 1:1 et 1:95, avantageusement entre 1:10 et 1: 50.

4. Composition pour son utilisation suivant l'une quelconque des revendications précédentes, **caractérisée en ce qu'**elle comprend au moins 2% en poids, avantageusement au moins 4% en poids de polymère silicone réticulé possédant des greffons hydrophile polypropylène glycol et/ou de polyéthylène glycol, par rapport au poids sec de la composition.

5. Composition pour son utilisation suivant l'une quelconque des revendications précédentes, caratérisée en ce qu'elle comprend une phase huile dans eau comprenant au moins le dérivé d'acide salicylique de formule I ou le mélange de dérivés d'acide salicylique de formule I dispersée dans une phase à base de silicone.

6. Composition pour son utilisation suivant l'une quelconque des revendications précédentes, **caractérisée en ce que** les cellules végétales dédifférenciées et élicitées de vigne, avant leur broyage éventuel, comprennent au moins 0,2% en poids de dérivé(s) d'acide salicylique de formule I, telle que défini(s) à la revendication 1.

7. Composition pour son utilisation l'une quelconque des revendications précédentes, **caractérisée en ce qu'**elle contient de 0,8% en poids à 5% en poids d'acide salicylique, par rapport au poids sec de la composition.

8. Composition pour son utilisation suivant l'une quelconque des revendications précédentes, **caractérisée en ce qu'**elle comprend des cellules végétales dédifférenciées élicitées de vigne non broyées.

9. Composition pour son utilisation suivant l'une quelconque des revendications précédentes, **caractérisée en ce qu'**elle comprend un broyat de cellules végétales dédifférenciées élicitées de vigne, ledit broyat comprenant au moins 95% en poids, avantageusement au moins 97% en poids des cellules végétales dédifférenciées élicitées avant leur broyage de vigne.

10. Composition pour son utilisation suivant l'une quelconque des revendications précédentes, **caractérisée en ce qu'**elle comprend en outre une ou des vitamines B, en particulier au moins de la vitamine B3.

11. Composition pour son utilisation suivant l'une quelconque des revendications précédentes, **caractérisée en ce qu'**elle se présente sous la forme d'une double émulsion dont les goutellettes à base d'eau et d'huile sont dispersées dans une phase à base de silicone et présentent une taille moyenne en nombre inférieure à 200µm, avantageusement comprise entre 50µm et 150µm.

12. Composition pour son utilisation suivant la revendication 11, **caractérisée en ce que** les gouttelettes à base d'eau et d'huile contiennent le dérivé d'acide salicylique de formule I ou un mélange de dérivés d'acide salicylique de formule I, et un composé basique pour régler le pH des gouttelettes entre 4 et 8, avantageusement entre 4,5 et 7, de préférence entre 4,5 et 6,5.

13. Composition pour son utilisation suivant la revendication 11 ou 12, **caractérisée en ce que** les gouttelettes à base d'eau et d'huile comprennent de l'urée et/ou de la bétaine.

14. Composition pour son utilisation suivant l'une des revendications 11 à 13, **caractérisée en ce que** les gouttelettes à base d'eau et d'huile comprennent au moins un agent tensio-actif et/ou un alcool.

15. Composition pour son utilisation suivant l'une des revendications 11 à 14, **caractérisée en ce que** la phase silicone comprend un mélange (a) de silicone(s) volatile(s) dont la pression de vapeur est comprise entre 1Pa et 2000Pa à 25°C et dont la viscosité à 25°C est comprise entre 0,1 et 10 centistokes, et (b) de silicone(s) linéaire(s) réticulé(s), le rapport en poids silicone(s) volatile(s) / silicone(s) linéaire(s) réticulé(s) étant compris entre 0,1 et 3, avantageusement entre 0,1 et 1.

16. Composition pour son utilisation suivant l'une quelconque des revendications précédentes, **caractérisée en ce qu'**elle se présente sous la forme d'une composition prête à l'application topique contenant de 0,5% en poids à 5% en poids d'un dérivé d'acide salicylique de formule I, avantageusement de 0,7 à 3 % en poids, de préférence de 0,9 à 2,5% en poids, par rapport au poids total de la composition.

17. Composition pour son utilisation suivant l'une quelconque des revendications précédentes pour son utilisation dans le traitement de dermatoses inflammatoires, en particulier de lésions érythémato-squameuses de psoriasis.

## Patentansprüche

1. Topische Zusammensetzung für die Verwendung zur Restrukturierung der Epidermis oder zur Behandlung von Dermatose durch topische Anwendung, die Folgendes umfasst (a) eine Öl-in-Wasser-Dispersion oder dafür ausgelegt, nach Zugabe einer Menge Wasser eine Öl-in-Wasser-Dispersion zu bilden, und (b) eine wirksame Menge mindestens eines Salicylsäurederivats der Formel I oder eines Gemischs von Salicylsäurederivaten der Formel I 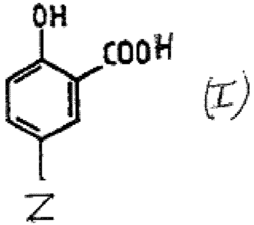 worin Z ein Wasserstoffatom oder ein Rest der folgenden Formel ist 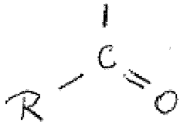 wobei R ein gerader oder verzweigter Alkylrest mit 1 bis 18 Kohlenstoffatomen, insbesondere mit 6 bis 14 Kohlenstoffatomen ist,
wobei die wirksame Menge mindestens größer als 0,5%, bezogen auf das Trockengewicht der Zusammensetzung, ist,
**dadurch gekennzeichnet, dass** sie außerdem mindestens 0,5 Gew.-% undifferenzierte und induzierte Pflanzenzellen von Wein oder ein Homogenat undifferenzierter und induzierter Pflanzenzellen von Wein in getrockneter Form, bezogen auf das Gesamtgewicht der Zusammensetzung, umfasst, wobei die undifferenzierten und induzierten Pflanzenzellen von Wein oder das Homogenat undifferenzierter und induzierter Pflanzenzellen von Wein mindestens 0,5 Gew.-% Stilbene, bezogen auf das Trockengewicht der Zellen, enthalten/enthält.

2. Zusammensetzung für die Verwendung nach Anspruch 1, **dadurch gekennzeichnet, dass** das Gewichtsverhältnis zwischen dem Trockengewicht des Salicylsäurederivats oder des Gemischs von Salicylsäurederivaten einerseits und dem Trockengewicht undifferenzierter und induzierter Pflanzenzellen von Wein oder eines Homogenats solcher undifferenzierter und induzierter Pflanzenzellen von Wein andererseits zwischen 1:50 und 50:1, vorteilhafterweise zwischen 1:25 und 40:1, vorzugsweise zwischen 1:1 und 30:1 beträgt.

3. Zusammensetzung für die Verwendung nach Anspruch 1 oder 2, **dadurch gekennzeichnet, dass** sie außerdem mindestens ein Silikonpolymer oder ein Silikonpolymergemisch umfasst, wobei das Gewichtsverhältnis zwischen dem Trockengewicht des Salicylsäurederivats der Formel I und dem Gemisch von Salicylsäurederivaten der Formel I einerseits und dem Silikonpolymer oder dem Gemisch von Silikonpolymeren andererseits zwischen 1:1 und 1:95, vorteilhafterweise zwischen 1:10 und 1:50 beträgt.

4. Zusammensetzung für die Verwendung nach einem der vorhergehenden Ansprüche, **dadurch gekennzeichnet, dass** sie mindestens 2 Gew.-%, vorteilhafterweise mindestens 4 Gew.-% vernetztes Silikonpolymer mit hydrophilen Polypropylenglykol- und/oder Polyethylenglykol-Pfropfungen, bezogen auf das Trockengewicht der Zusammensetzung, umfasst.

5. Zusammensetzung für die Verwendung nach einem der vorhergehenden Ansprüche, **dadurch gekennzeichnet, dass** sie eine Öl-in-Wasser-Phase umfasst, die mindestens das Salicylsäurederivat der Formel I oder das Gemisch von Salicylsäurederivaten der Formel I, dispergiert in einer Phase auf der Basis von Silikon, umfasst.

6. Zusammensetzung für die Verwendung nach einem der vorhergehenden Ansprüche, **dadurch gekennzeichnet, dass** die undifferenzierten und induzierten Pflanzenzellen von Wein vor ihrer optionalen Homogenisation mindestens 0,2 Gew.-% Salicylsäurederivat(e) der Formel I nach Anspruch 1 umfassen.

7. Zusammensetzung für die Verwendung nach einem der vorhergehenden Ansprüche, **dadurch gekennzeichnet, dass** sie 0,8 Gew.-% bis 5 Gew.-% Salicylsäure, bezogen auf das Trockengewicht der Zusammensetzung, enthält.

8. Zusammensetzung für die Verwendung nach einem der vorhergehenden Ansprüche, **dadurch gekennzeichnet, dass** sie undifferenzierte induzierte, nicht homogenisierte Pflanzenzellen von Wein umfasst.

9. Zusammensetzung für die Verwendung nach einem der vorhergehenden Ansprüche, **dadurch gekennzeichnet, dass** sie ein Homogenat undifferenzierter induzierter Pflanzenzellen von Wein umfasst, wobei das Homogenat mindestens 95 Gew.-%, vorteilhafterweise mindestens 97 Gew.-% undifferenzierte induzierte Pflanzenzellen von Wein vor deren Homogenisierung umfasst.

10. Zusammensetzung für die Verwendung nach einem der vorhergehenden Ansprüche, **dadurch gekennzeichnet, dass** sie außerdem ein B-Vitamin oder B-Vitamine, insbesondere mindestens Vitamin B3, umfasst.

11. Zusammensetzung für die Verwendung nach einem der vorhergehenden Ansprüche, **dadurch gekennzeichnet, dass** sie in Form einer Doppelemulsion vorliegt, deren Tröpfchen auf der Basis von Wasser und Öl in einer Phase auf der Basis von Silikon dispergiert sind und eine mittlere Größe unter 200 µm, vorteilhafterweise zwischen 50 µm und 150 µm aufweisen.

12. Zusammensetzung für die Verwendung nach Anspruch 11, **dadurch gekennzeichnet, dass** die Tröpfchen auf der Basis von Wasser und Öl das Salicylsäurederivat der Formel I oder ein Gemisch von Salicylsäurederivaten der Formel I und eine basische Verbindung zur Einstellung des pH-Werts der Tröpfchen auf zwischen 4 und 8, vorteilhafterweise zwischen 4,5 und 7, vorzugsweise zwischen 4,5 und 6,5 enthalten.

13. Zusammensetzung für die Verwendung nach Anspruch 11 oder 12, **dadurch gekennzeichnet, dass** die Tröpfchen auf der Basis von Wasser und Öl Harnstoff und/oder Betain umfassen.

14. Zusammensetzung für die Verwendung nach einem der Ansprüche 11 bis 13, **dadurch gekennzeichnet, dass** die Tröpfchen auf der Basis von Wasser und Öl mindestens ein Tensid und/oder einen Alkohol umfassen.

15. Zusammensetzung für die Verwendung nach einem der Ansprüche 11 bis 14, **dadurch gekennzeichnet, dass** die Silikonphase ein Gemisch von (a) flüchtige(m/n) Silikon(en) mit einem Dampfdruck zwischen 1 Pa und 2000 Pa bei 25°C und mit einer Viskosität bei 25°C zwischen 0,1 und 10 centistokes und (b) gerade(m/n) vernetzte(m/n) Silikon(en) umfasst, wobei das Gewichtsverhältnis von flüchtige(m/n) Silikon(en)/gerade(m/n) vernetzte(m/n) Silikon(en) zwischen 0,1 und 3, vorteilhafterweise zwischen 0,1 und 1 beträgt.

16. Zusammensetzung für die Verwendung nach einem der vorhergehenden Ansprüche, **dadurch gekennzeichnet, dass** sie in Form einer für die topische Anwendung gebrauchsfertigen Zusammensetzung vorliegt, die 0,5 Gew.-% bis 5 Gew.-% eines Salicylsäurederivats der Formel I, vorteilhafterweise 0,7 bis 3 Gew.-%, vorzugsweise 0,9 bis 2,5%, bezogen auf das Gesamtgewicht der Zusammensetzung, enthält.

17. Zusammensetzung für die Verwendung nach einem der vorhergehenden Ansprüche zur Verwendung bei der Behandlung entzündlicher Dermatosen, insbesondere von erythemato-squamösen Läsionen von Psoriasis.

## Claims

1. A topical composition for its use in the restructuring of the epidermis or in the treatment of dermatosis by topical application comprising (a) an oil-in-water dispersion or being adapted to form an oil-in-water dispersion after addition of a quantity of water, and (b) an effective amount of at least one salicylic acid derivative of the formula I or a mixture of salicylic acid derivatives of the formula I 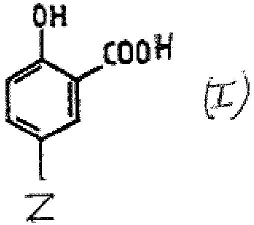 in which Z is a hydrogen atom or a radical of formula 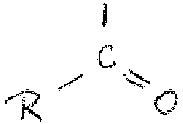 with R a linear or branched alkyl radical with 1 to 18 carbon atoms, in particular with 6 to 14 carbon atoms,
said effective amount being at least greater than 0.5% by dry weight of the composition,
**characterised in that** it further comprises at least 0.5% by weight of dedifferentiated and elicited vine plant cells or of a grinding of dedifferentiated and elicited vine plant cells in dry form relative to the total weight of the composition, said dedifferentiated and elicited vine plant cells or said grinding of dedifferentiated and elicited vine plant cells containing at least 0.5% by weight of stilbenes relative to the dry weight of the cells.

2. Composition for its use according to Claim 1, **characterised in that** the ratio by weight between on the one hand, the dry weight of salicylic acid derivative or the mixture of salicylic derivatives and, on the other hand, the dry weight of dedifferentiated and elicited vine plant cells or a grinding of such dedifferentiated and elicited vine plant cells is between 1:50 and 50:1, advantageously between 1:25 and 40:1, preferably between 1:1 and 30:1.

3. Composition for its use according to Claim 1 or 2, **characterised in that** it also comprises at least one silicone polymer or silicone polymer mixture, the ratio by weight of, on the one hand, the dry weight of salicylic acid derivative of formula I and the mixture of salicylic acid derivatives of formula I, and on the other hand, the silicone polymer or silicone polymer mixture being between 1:1 and 1:95, advantageously between 1:10 and 1:50.

4. Composition for its use according to any one of the preceding claims, **characterised in that** it comprises at least 2% by weight, advantageously at least 4% by weight of cross-linked silicone polymer having hydrophilic polypropylene glycol and/or polyethylene glycol grafts, relative to the dry weight of the composition.

5. A composition for its use according to any one of the preceding claims, **characterised in that** it comprises an oil-in-water phase comprising at least the salicylic acid derivative of formula I or the mixture of salicylic acid derivatives of formula I dispersed in a silicone based phase.

6. Composition for its use according to any one of the preceding claims, **characterised in that** the dedifferentiated and elicited vine plant cells, before their optional grinding, comprise at least 0.2% by weight of salicylic acid derivative (s) of formula I, as defined (s) in claim 1.

7. Composition for its use according to any one of the preceding claims, **characterised in that** it contains from 0.8% by weight to 5% by weight of salicylic acid, relative to the dry weight of the composition.

8. Composition for its use according to any one of the preceding claims, **characterised in that** it comprises ungrounded, dedifferentiated and elicited vine plant cells.

9. Composition for its use according to any one of the preceding claims, **characterised in that** it comprises a grinding of dedifferentiated and elicited vine plant cells, said grinding comprising at least 95% by weight, advantageously at least 97% by weight of the dedifferentiated and elicited vine plant cells before grinding.

10. Composition for its use according to any one of the preceding claims, **characterised in that** it further comprises one or more vitamins B, in particular at least vitamin B3.

11. Composition for its use according to any one of the preceding claims, **characterised in that** it has the form of a double emulsion whose water and oil based droplets are dispersed in a silicone based phase and have a average size in number less than 200µm, advantageously between 50µm and 150µm.

12. A composition for its use according to claim 11, **characterised in that** the water and oil based droplets contain the salicylic acid derivative of the formula I or a mixture of salicylic acid derivatives of the formula I, and a basic compound for regulating the pH of the droplets between 4 and 8, advantageously between 4.5 and 7, preferably between 4.5 and 6.5.

13. Composition for its use according to claim 11 or 12, **characterised in that** the water and oil based droplets comprise urea and/or betaine.

14. Composition for its use according to any one of the claims 11 to 13, **characterised in that** the water and oil based droplets comprise at least a surfactant and/or an alcohol.

15. Composition for its use according to any one of the claims 11 to 14, **characterised in that** the silicone phase comprises a mixture (a) of volatile silicone (s) whose vapour pressure is between 1 Pa and 2000 Pa at 25° C. and whose viscosity at 25° C is between 0.1 and 10 centistokes, and (b) cross-linked linear silicone(s), the ratio by weight volatile silicone(s) / cross-linked linear silicone(s) being between 0.1 and 3, preferably between 0.1 and 1.

16. Composition for its use according to any one of the preceding claims, **characterised in that** it has the form of a composition ready for topical application containing from 0.5% by weight to 5% by weight of a derivative of salicylic acid of formula I, advantageously from 0.7 to 3% by weight, preferably from 0.9 to 2.5% by weight, relative to the total weight of the composition.

17. Composition for its use according to any one of the preceding claims, for its use in the treatment of inflammatory dermatosis, in particular erythemato-squameusic lesions of psoriasis.
